## Europäisches Patentamt

## European Patent Office

## Office européen des brevets

(11) Veröffentlichungsnummer: **0 003 362**
**B1**

(12) **EUROPÄISCHE PATENTSCHRIFT**

(45) Veröffentlichungstag der Patentschrift:
14.10.81

(21) Anmeldenummer: 79100253.8

(22) Anmeldetag: 29.01.79

(51) Int. Cl.³: **C 04 B 13/28**, C 07 G 1/00

(54) Fliessmittel für Beton und Mörtel und Verfahren zu seiner Herstellung.

(30) Priorität: 30.01.78 DE 2803923
14.03.78 DE 2811098

(43) Veröffentlichungstag der Anmeldung:
08.08.79 Patentblatt 79/16

(45) Bekanntmachung des Hinweises auf die Patenterteilung:
14.10.81 Patentblatt 81/41

(84) Benannte Vertragsstaaten:
BE CH FR GB IT LU NL SE

(56) Entgegenhaltungen:
**AU-B-447 431**
**DE-A-2 616 170**
**Fr-A-2 038 666**
**US-A-3 931 072**
**US-A-4 054 462**

(73) Patentinhaber: **Holmen GmbH, DEA-Scholven-Strasse,
D-7500 Karlsruhe 21 (DE)**

(72) Erfinder: **Köhler, Leo, Beethovenstraße 32,
D-6712 Bobenheim (DE)**

(74) Vertreter: **Lamprecht, Helmut, Dipl.-Ing.,
Corneliusstrasse 42, D-8000 München 5 (DE)**

## Fließmittel für Beton und Mörtel und Verfahren zu seiner Herstellung

Die Erfindung betrifft ein Fließmittel für Beton und Mörtel auf der Basis von Ligninsulfonat einerseits und einem kernsulfonierten oder sulfomethylierten, anschließend kondensierten Aromaten aus der Gruppe Phenol, Kresol, Naphthalin andererseits, sowie ein Verfahren zur Herstellung eines solchen Fließmittels.

Aufgabe der Erfindung ist es, einerseits eine hohe Fließfähigkeit des Betons zu erreichen, andererseits aber trotz hoher Fließfähigkeit die Abbindezeiten nicht unerwünscht zu verlängern.

Die Verflüssigung von Beton mit Ligninsulfonaten und anderen Dispergatoren ist bekannt. In der Fertigteiltechnik und bei Transportbeton bzw. armierten Betonbauten hat man sogenannte Superverflüssiger eingesetzt, um eine besonders gute Fließfähigkeit zu erlangen. Für diesen Zweck haben sich bisher Ligninsulfonate als ungeeignet erwiesen. Während auf anderen, begrenzten Einsatzgebieten mit einer relativ geringen Dosierung von Ligninsulfonaten deren gute Dispergierwirkung ohne besondere Nachteile nutzbar gemacht werden konnte, zeigte sich bei einer höheren Dosierung zwar eine bessere Verflüssigung, der Beton blutete jedoch aus und die Abbindezeiten verschoben sich in unerwünschte Dimensionen.

Um die gute Dispergierwirkung der Ligninsulfonate nutzbar zu machen, hat man alle möglichen Mischungen von Ligninsulfonaten mit anorganischen und organischen Salzen (z. B. Dikarbonsäuren) versucht, um bessere Eigenschaften zu erzielen. Da diese Versuche erfolglos blieben, wurden synthetische Dispergatoren angewandt, die aus dem Bereich der kondensierten Aromaten, kondensierten Heterozyklen und Aminoverbindungen stammen. Mit diesen Mitteln ergaben sich keine unerwünschten Abbindezeiten, jedoch konnte auch mit diesen Mitteln das Ausbluten des Betons nicht ganz vermieden werden. Zudem waren auch diese keineswegs voll befriedigenden Mittel im Vergleich zu reinen Ligninsulfonaten und Derivaten viel zu teuer.

Der Erfindung liegt die Aufgabe zugrunde, ein Fließmittel auf der Basis von Ligninsulfonat zu schaffen, das preiswert ist und alle betontechnologischen Anforderungen bezüglich Verflüssigung, Abbindeverhalten und Dosierung erfüllt und das andererseits besonders kostengünstig herstellbar ist. Außerdem ist es eine Aufgabe der Erfindung, ein vorteilhaft durchführbares Verfahren für die Herstellung eines solchen Fließmittels auf der Basis von Ligninsulfonat zu schaffen.

Die Lösung der gestellten Aufgabe nach Schaffung eines Fließmittels besteht darin, daß bei dem anfangs erwähnten Fließmittel das Reaktionsgemisch der Basisstoffe kondensiert und sulfitiert ist.

Ein derartiges Fließmittel ist preiswert und erfüllt trotzdem alle Anforderungen hinsichtlich Verflüssigung, Abbindeverhalten und Dosierung.

Ein Verfahren zur Herstellung dieses erfindungsgemäßen Fließmittels für Beton und Mörtel auf der Basis von Ligninsulfonat einerseits und einem kernsulfonierten, anschließend kondensierten Aromaten aus der Gruppe Phenol, Kresol, Naphthalin andererseits, besteht darin, daß der durch die Kernsulfonierung wasserlöslich gemachte Aromat anschließend mit Harnstoff und Formaldehyd kondensiert und dieses Kondensat mit Ligninsulfonat unter weiterer Zugabe von Harnstoff und Formaldehyd kondensiert und schließlich mit Natriumsulfit sulfitiert wird.

Ausgehend von den gleichen Basisstoffen besteht ein anderes Verfahren zur Herstellung des erfindungsgemäßen Fließmittels für Beton und Mörtel darin, daß der durch die Kernsulfonierung wasserlöslich gemachte Aromat anschließend mit Harnstoff und Formaldehyd kondensiert, dann sulfitiert und anschließend mit Formaldehyd und Hrnstoff kondensiert, wobei Ligninsulfonat zugegeben wird, dabei kann das Ligninsulfonat entweder nach dem Sulfitieren des kernsulfonierten Aromaten oder aber nach diesem Sulfitieren und dem anschließenden Nachkondensieren mit Formaldehyd zugegeben werden.

Ein weiteres Verfahren zur Herstellung des erfindungsgemäßen Fließmittels auf der Basis von Ligninsulfonat einerseits und einem sulfomethylierten, anschließend kondensierten Aromaten aus der Gruppe Phenol, Kresol, Naphthalin andererseits besteht darin, daß der Aromat unter Zugabe von Natriumsulfit und Wasser sulfitiert und anschließend mit Formaldehyd kondensiert wird, und daß dieses Kondensat mit Ligninsulfonat, Harnstoff und Formaldehyd kondensiert und mit Natriumsulfit sulfitiert wird.

Für die Zwecke der Erfindung können vergorene oder unvergorene Ligninsulfonate eingesetzt werden, aber auch Ligninsulfonate, bei welchen der in ihnen enthaltene Zucker durch alkalische Oxydation zerstört worden ist.

Die Ligninlauge kann als Kation Calzium, Magnesium, Aluminium, Natrium oder Ammonium enthalten, wobei der Kationenaustausch vor der Zugabe des Ligninsulfonats durch Umsalzen durchgeführt werden kann.

Vorzugsweise wird das erfindungsgemäße Verfahren bei einem pH-Wert von 4 − 8 durchgeführt.

Das erfindungsgemäß hergestellte Fließmittel verleiht dem Beton oder Mörtel eine hohe Fließfähigkeit, ohne zu unerwünschtem Ausbluten oder zu unerwünschten Abbindezeiten zu führen. Dieses Fließmittel ist überdies im Vergleich mit bekannten, ihm hinsichtlich der vorteilhaften Eigenschaften am nächsten kommenden Fließmitteln wesentlich preisgünstiger, wobei insbesondere die Möglichkeit, bei der Herstellung einen höheren Anteil an Ligninsulfonat zu verwenden, bei mindestens gleicher Wirkung zu einem beachtlichen Preisvorteil führt.

Anhand der nun folgenden Beschreibung einiger Ausführungsbeispiele der Erfindung wird diese näher erläutert.

In kernsulfonierter Form kann beispielsweise beim erfindungsgemäßen Verfahren Phenol, Naphthalin, Kresol oder eine beliebige Mischung dieser drei Aromaten umgesetzt werden.

### A. Herstellung des Fließmittels auf der Basis von Ligninsulfonat und kernsulfoniertem Aromat

Das gewählte Aromat wird mit Schwefelsäure solange sulfoniert, bis es klar wasserlöslich ist, dann wird mit Harnstoff und Formaldehyd kondensiert. Dem Kondensat werden zur Nachkondensation Ligninsulfonat, Harnstoff und Formadehyd zugegeben, danach wird mit Natriumsulfit sulfitiert.

### Beispiel I

100 Teile Phenol und 120 Teile konzentrierte Schwefelsäure werden bei 115°C 2 Stunden im Autoklav sulfoniert, dann wird abgekühlt auf ca. 50°C;

Kondensation:
30 Teile Harnstoff ⎱ 2 Stunden bei
70−80 Teile Wasser ⎰ 50°C;
100 Teile Formaldehyd 30%ig zugeben, 12 Stunden bei 30−50°C weiter kondensieren;
Zugabe:
400−500 Teile Lignin-Sulfonat 45%ig, 30 Minuten kondensieren bei 30−80°C;
Zugabe:
20−50 Teile Harnstoff, 30 Minuten bei 30−80°C kondensieren;
Zugabe:
50−100 Teile Formaldehyd 30%ig, 30 Minuten kondensieren bei 30−80°C;
Zugabe:
30−60 Teile Natriumsulfit, 30 Minuten sulfitieren bei 30−80°C;
Zugabe:
Wasser, bis gewünschte Trockensubstanz erreicht ist.

Der pH-Wert wird auf 4−8 eingestellt. Es handelt sich bei diesem wie auch bei den folgenden Beispielen jeweils um Gewichtsteile.

Das gewählte Aromat wird mit Schwefelsäure so lange sulfoniert, bis es klar wasserlöslich ist, dann wird mit Harnstoff und Formaldehyd kondensiert. Danach wird nach einer Variante des Verfahrens mit Natriumsulfit sulfitiert und anschließend nach Zugabe des Ligninsulfonats mit Formaldehyd und Harnstoff nachkondensiert.

### Beispiel II

100 Gewichtsteile Phenol und 120 Teile konzentrierte Schwefelsäure werden bei 115°C 2 Stunden im Autoklaven sulfoniert, dann wird abgekühlt auf ca. 50°C;

Kondensation:
30 Teile Harnstoff ⎱ 2 Stunden bei
70−80 Teile Wasser ⎰ 50°C;
100 Teile Formaldehyd 30%ig zugeben, 12 Stunden bei 30−50°C weiter kondensieren;
nun auf 80°C aufheizen und mit 30−60 Teilen Natriumsulfit 1 Stunde sulfitieren;
Zugabe:
100−300 Teile Formaldehyd 30%ig, 60 Minuten kondensieren bei 50−80°C;
Zugabe:
400−800 Teile Ligninsulfonat 45%ig, 60 Minuten kondensieren bei 50−80°C;
Zugabe:
20−50 Teile Harnstoff, 60 Minuten kondensieren bei 50−80°C;
Zugabe:
Wasser, bis die gewünschte Trockensubstanz erreicht ist.

Der pH-Wert wird auf 4−8 eingestellt.

### Beispiel III

100 Teile Phenol und 120 Teile konzentrierte Schwefelsäure werden bei 115°C 2 Stunden im Autoklaven sulfoniert, dann wird abgekühlt auf ca. 50°C;

Kondensation:
30 Teile Harnstoff ⎱ 2 Stunden bei
70−80 Teile Wasser ⎰ 50°C;
100 Teile Formaldehyd 30%ig zugeben, 12 Stunden bei 30−50°C weiter kondensieren;
nun auf 80°C aufheizen und mit 30−60 Teilen Natriumsulfat 1 Stunde sulfitieren;
Zugabe:
400−800 Teile Ligninsulfonat 45%ig, 60 Minuten kondensieren bei 50−80°C;
Zugabe:
100−300 Teile Formaldehyd 30%ig, 60 Minuten kondensieren bei 50−80°C;
Zugabe:
20−50 Teile Harnstoff, 60 Minuten kondensieren bei 50−80°C.

Der pH-Wert wird auf 4−8 eingestellt.

### B. Herstellung des Fließmittels auf der Basis von Ligninsulfonat und sulfomethyliertem Aromat

Das gewählte Aromat wird mit Natriumsulfit und Wasser sulfitiert, anschließend wird mit Formaldehyd kondensiert. Nun wird Ligninsulfonat zugegeben, mit Harnstoff und Formaldehyd nachkondensiert und mit Natriumsulfit nachsulfitiert.

Beispiel IV

100 Teile Phenol und 40 Teile Natriumsulfit und 30 Teile Wasser werden 1 Stunde lang bei 100°C sulfitiert; dann erfolgt die Zugabe von 100 Teilen 30%igem Formaldehyd und es wird 2,5 Stunden bei 105°C kondensiert:

nach Abdestillieren von 40 Teilen Wasser wird abgekühlt und anschließend verdünnt;
Zugabe:
 200 – 300 Teile Lignin-Sulfonat 45%ig, 30 Minuten rühren bei 30 – 80°C;
Zugabe:
 20 – 50 Teile Harnstoff, 30 Minuten kondensieren bei ca. 30 – 80°C;
Zugabe:
 50 – 100 Teile Formaldehyd 30%ig, 30 Minuten kondensieren bei 30 – 80°C;
Zugabe:
 30 – 60 Teile Natriumsulfit, 30 Minuten sulfitieren bei 30 – 80°C;
Zugabe:
 Wasser, bis gewünschte Trockensubstanz erreicht ist.

Der pH-Wert wird auf 4 – 8 eingestellt. Das Ligninsulfonat kann beim erfindungsgemäßen Verfahren als vergorene bzw. zuckerfreie Lauge zugegeben werden, die als Kation Calzium, Magnesium, Aluminium, Natrium oder Ammonium enthält.

**Patentansprüche**

1. Fließmittel für Beton und Mörtel auf der Basis von Ligninsulfonat einerseits und einem kernsulfonierten oder sulfomethylierten, anschließend kondensierten Aromaten aus der Gruppe Phenol, Kresol, Naphthalin andererseits, dadurch gekennzeichnet, daß das Reaktionsgemisch der Basisstoffe kondensiert und sulfitiert ist.

2. Verfahren zur Herstellung eines Fließmittels für Beton und Mörtel auf der Basis von Ligninsulfonat einerseits und einem kernsulfonierten, anschließend kondensierten Aromaten aus der Gruppe Phenol, Kresol, Naphthalin andererseits, dadurch gekennzeichnet, daß der durch die Kernsulfonierung wasserlöslich gemachte Aromat anschließend mit Harnstoff und Formaldehyd kondensiert und dieses Kondensat mit Ligninsulfonat unter weiterer Zugabe von Harnstoff und Formaldehyd kondensiert und schließlich mit Natriumsulfit sulfitiert wird.

3. Verfahren zur Herstellung eines Fließmittels für Beton und Mörtel auf der Basis von Ligninsulfonat einerseits und einem sulfomethylierten, anschließend kondensierten Aromaten aus der Gruppe Phenol, Kresol, Naphthalin andererseits, dadurch gekennzeichnet, daß der Aromat unter Zugabe von Natriumsulfit und Wasser sulfitiert und anschließend mit Formaldehyd kondensiert wird, und daß dieses Kondensat mit Ligninsulfonat, Harnstoff und Formaldehyd kondensiert und mit Natriumsulfit sulfitiert wird.

4. Verfahren zur Herstellung eines Fließmittels für Beton und Mörtel auf der Basis von Ligninsulfonat einerseits und einem kernsulfonierten, anschließend kondensierten Aromaten aus der Gruppe Phenol, Kresol, Naphthalin andererseits, dadurch gekennzeichnet, daß der durch die Kernsulfonierung wasserlöslich gemachte Aromat anschließend mit Harnstoff und Formaldehyd kondensiert, dann sulfitiert und anschließend mit Formaldehyd und Harnstoff kondensiert wird, wobei Ligninsulfonat zugegeben wird.

5. Verfahren nach Anspruch 4, dadurch gekennzeichnet, daß nach dem Sulfitieren des kernsulfonierten Aromats mit Formaldehyd nachkondensiert, dann Ligninsulfonat zugegeben und schließlich mit Harnstoff weiterkondensiert wird.

6. Verfahren nach Anspruch 4, dadurch gekennzeichnet, daß nach dem Sulfitieren des kernsulfonierten Aromats Ligninsulfonat zugegeben, dann mit Formaldehyd nachkondensiert und schließlich mit Harnstoff weiter kondensiert wird.

7. Verfahren nach einem der Ansprüche 2 bis 6, dadurch gekennzeichnet, daß vergorenes Ligninsulfonat zugegeben wird.

8. Verfahren nach einem der Ansprüche 2 bis 6, dadurch gekennzeichnet, daß unvergorenes Ligninsulfonat zugegeben wird.

9. Verfahren nach einem der Ansprüche 2 bis 5, dadurch gekennzeichnet, daß vor der Zugabe des Ligninsulfonats der in ihm enthaltene Zucker durch alkalische Oxidation zerstört wird.

10. Verfahren nach einem der Ansprüche 2 bis 9, dadurch gekennzeichnet, daß die Ligninsulfonatlauge als Kation ein aus der Gruppe Calzium, Magnesium, Aluminium, Natrium, Ammonium ausgewähltes Kation enthält.

11. Verfahren nach einem der Ansprüche 2 bis 10 , dadurch gekennzeichnet, daß es überwiegend bei einem pH-Wert von 4—8 durchgeführt wird.

12. Verfahren nach Anspruch 2, dadurch gekennzeichnet, daß 100 Gewichtsteile Phenol mit 120 Gewichtsteilen konzentrierter Schwefelsäure bei 115°C zwei Stunden im Autoklaven sulfoniert werden, worauf Abkühlung auf 50°C stattfindet, daß die Kondensation mit 30 Gewichtsteilen Harnstoff und 70—80 Gewichtsteilen Wasser 2 Stunden bei 50°C erfolgt, dann 100 Gewichtsteile 30%iges Formaldehyd zugegeben werden und bei 30—50°C 12 Stunden weiterkondensiert wird, daß 400—500 Gewichtsteile 45%iges Ligninsulfonat zugegeben werden, daß 30 Minuten bei 30—80°C gerührt wird, dann 20—50 Gewichtsteile Harnstoff zugegeben werden, 30 Minuten bei 30—80°C kondensiert werden, dann 50—100 Gewichtsteile 30%iges Formaldehyd zugegeben werden, 30 Minuten bei 30—80°C kondensiert wird, worauf schließlich bis zur Erreichung der gewünschten Trockensubstanz Wasser zugegeben und der pH-Wert auf 4—8 eingestellt wird.

13. Verfahren nach Anspruch 5, dadurch gekennzeichnet, daß 100 Gewichtsteile Phenol mit 120 Gewichtsteilen konzentrierter Schwefelsäure bei 115°C zwei Stunden im Autoklaven sulfoniert werden, worauf Abkühlung auf 50°C stattfindet, daß die Kondensation mit 30 Gewichtsteilen Harnstoff und 70—80 Gewichtsteilen Wasser 2 Stunden bei 50°C erfolgt, dann 100 Gewichtsteile 30%iges Formaldehyd zugegeben werden und bei 30—50°C 12 Stunden weiterkondensiert wird, daß anschließend auf 80°C aufgeheizt und mit 30—60 Gewichtsteilen Natriumsulfit 1 Stunde sulfitiert wird, daß dann 100—300 Gewichtsteile 30%iges Formaldehyd zugegeben werden, daß 60 Minuten bei 50—80°C gerührt wird, dann 400—800 Gewichtsteile 45%iges Ligninsulfonat zugegeben werden und 60 Minuten bei 50—80°C kondensiert wird, daß dann 20—50 Gewichtsteile Harnstoff zugegeben werden, 60 Minuten bei 50—80°C kondensiert wird und dann bis zur Erreichung der gewünschten Trockensubstanz Wasser zugegeben und der pH-Wert auf 4—8 eingestellt wird.

14. Verfahren nach Anspruch 3, dadurch gekennzeichnet, daß 100 Gewichtsteile Phenol und 40 Gewichtsteile Natriumsulfit mit 30 Gewichtsteilen Wasser eine Stunde lang bei 100°C sulfitiert werden, daß dann 100 Gewichtsteile 30%iges Formaldehyd zugegeben werden und 2,5 Stunden bei 105°C kondensiert wird, daß nach dem Abdestillieren von 40 Gewichtsteilen Wasser abgekühlt und anschließend verdünnt wird, daß dann 200—300 Gewichtsteile 45%iges Ligninsulfonat zugegeben werden, daß 30 Minuten bei 30—80°C gerührt wird, daß dann 20—50 Gewichtsteile Harnstoff zugegeben werden, 30 Minuten bei ca. 30—80°C kondensiert wird, dann 50—100 Gewichtsteile 30%iges Formaldehyd zugegeben werden, 30 Minuten bei 30—80°C kondensiert wird und schließlich 30—60 Gewichtsteile Natriumsulfit zugegeben werden, 30 Minuten bei 30—80°C sulfitiert wird und bis zur Erreichung der gewünschten Trockensubstanz Wasser zugegeben und der pH-Wert auf 4—8 eingestellt wird.

15. Verfahren nach Anspruch 6, dadurch gekennzeichnet, daß 100 Gewichtsteile Phenol mit 120 Gewichtsteilen konzentrierter Schwefelsäure bei 115°C zwei Stunden lang im Autoklaven sulfoniert werden, worauf Abkühlung auf 50°C stattfindet, daß die Kondensation mit 30 Gewichtsteilen Harnstoff und 70—80 Gewichtsteilen Wasser 2 Stunden bei 50°C erfolgt, dann 100 Gewichtsteile 30%iges Formaldehyd zugegeben werden und bei 30—50°C 12 Stunden lang weiterkondensiert wird, daß anschließend auf 80°C aufgeheizt und mit 30—60 Gewichtsteilen Natriumsulfit 1 Stunde sulfitiert wird, daß dann 400—800 Gewichtsteile 45%iges Ligninsulfonat zugegeben werden, daß 60 Minuten bei 50—80°C gerührt wird, daß dann 100—300 Teile 30%iges Formaldehyd zugegeben werden und 60 Minuten bei 50—80°C kondensiert wird, dann 20—50 Teile Harnstoff zugegeben werden und 60 Minuten bei 50—80°C kondensiert wird, worauf schließlich bis zur Erreichung der gewünschten Trockensubstanz Wasser zugegeben und der pH-Wert auf 4—8 eingestellt wird.

## Claims

1. A flowing agent for concrete and mortar, based on the one hand, on lignin sulfonate and, on the other hand, on a ring-sulfonated or sulfomethylated and thereafter condensed aromatic compound which is a member of the group consisting of phenol, cresol, naphthalene, characterized in that the mixture of the basic ingredients is condensed and sulfitized.

2. A process for producing a flowing agent for concrete and mortar, based on the one hand, on lignin sulfonate and, on the other hand, on a ringsulfonated or sulfomethylated and thereafter condensed aromatic compound which is a member of the group consisting of phenol, cresol, naphthalene, characterized in that the aromatic compound which has been made water-soluble by the ring-sulfonation thereafter is being condensed with the addition of urea and formaldehyde, and that the so formed condensate is being condensed with the addition of lignin sulfonate and with the further addition of urea and formaldehyde and that finally sulfitation is being effected by adding sodium sulphite.

3. A process for producing a flowing agent for concrete and mortar, based on the one band, on lignin sulfonate and, on the other hand, on a ring-sulfonated or sulfomethylated and thereafter condensed aromatic compound which is a member of the group consisting of phenol, cresol, naphthalene, characterized in that the aromatic compound is being subjected to sulfitation by adding water and sodium sulfite thereto, and thereafter is being condensed with the addition of formaldehyde, and that the so formed condensate is being condensed with the addition of lignin sulfonate, urea and formaldehyde, and that sulfitation is being effected by adding sodium sulfite.

4. A process for producing a flowing agent for concrete and mortar, based on the one hand, on lignin sulfonate and, on the other hand, on a ring-sulfonated or sulfomethylated and thereafter condensed aromatic compound which is a member of the group consisting of phenol, cresol, naphthalene, characterized in that the aromatic compound which has been made water-soluble by the ring-sulfonation thereafter is being condensed with the addition of urea and formaldehyde, that thereafter the so formed condensate is being sulfitized, and that subsequently, condensation with the addition of formaldehyde, urea and lignin sulfonate is being effected.

5. The process according to claim 4, characterized in that sulfitizing the ring-sulfonated aromatic is followed by the steps of after-condensing with formaldehyde, adding lignin sulfonate, and finally treating with urea for further condensation.

6. The process according to claim 4, charaterized in that sulfitizing the ring-sulfonated aromatic is followed by the steps of adding lignin sulfonate after-condensing with formaldehyde and treating with urea for further condensation.

7. The process according to one of claims 2 to 6, characterized in that fermented lignin sulfonate is being added.

8. The process according to one of claims 2 to 6, characterized in that unfermented lignin sulfonate is being added.

9. The process according to one of claims 2 to 5, characterized in that before adding lignin sulfonate, the sugar contained therin is destroyed by alkaline oxidation.

10. The process according to one claims 2 to 9, characterized in that lignin sulfonate liquor contains as cation a member of the group consisting of calcium, magnesium, aluminium, sodium, and ammonium.

11. The process according to one of claims 2 to 10, characterized in that the pH-value at which the process predominantly is carried out, is adjusted to between 4—8.

12. The process according to claim 2, characterized by sulfonating 100 parts by weight phenol with 120 parts by weight of concentrated sulfuric acid at 115°C for two hours in an autoclave, then cooling to 50°C, condensing with 30 parts by weight urea and 70—80 parts water for two hours at 50°C, then adding 100 parts by weight 30% formaldehyde and continuing condensation for 12 hours at 30—50°C, adding 400—500 parts by weight of 45% lignin, sulfonate, stirring for 30 min. at 30—80°C, then adding 20—50 parts by weight urea, condensing 30 min. at 30—80°C then adding 50—100 by weight parts 30% formaldehyde, condensing 30 min. at 30—80°C whereupon finally adding water for obtaining the desired dry substance, and adjusting the pH to 4—8.

13. The process according to claim 5, characterized by sulfonating 100 parts phenol by weight with 120 parts by weight concentrated sulfuric acid at 115°C for two hours in an autoclave, then cooling to 50°C, thereupon condensing with 30 parts by weight urea and 70—80 parts by weight water for zwo hours at 50°C, thereupon adding 100 parts by weight 30% formaldehyde and continuing the condensation for 12 hours at 30—50°C, subsequently heating up to 80°C and sulfitizing for one hour with 30—60 parts by weight sodium sulfite, then adding 100—300 parts by weight 30% formaldehyde and stirring for 60 minutes at 50—80°C, thereupon adding 400—800 parts by weight 45% lignin sulfonate and condensing for 60 minutes at 50—80°C, then adding 20—50 parts by weight urea and condensing for 60 minutes at 50—80°C, finally adding water to obtain the desired dry substance while adjusting the pH to 4—8.

14. The process according to claim 3, characterized by sulfitizing 100 parts by weight phenol with 40 parts by weight sodium sulfite and 30 parts by weight water for one hour at 100°C, then adding 100 parts by weight 30% formaldehyde and condensing for 2,5 hours at 105°C, distilling 40 parts by weight water colling and diluting, then adding 200—300 parts by weight 45% lignin sulfonate, stirring for 30 minutes at 30—80°C, thereafter adding 20—50 parts by weight urea condensing 30 minutes at about 30-80°C, thereafter adding 50—100 parts by weight 30% formaldehyde, condensing 30 minutes at 30—80°C, and finally adding 30—60 parts by weight sodium sulfite, sulfitizing 30 minutes at 30—80°C and adding water until the desired dry substance is obtained, and adjusting the pH to 4—8.

15. The process according to claim 6 characterized by sulfonating 100 parts by weight phenol with 120 parts by weight concentrated sulfuric acid for two hours at 115°C in an autoclave, whereupon cooling to 50°C occurs, then condensing with 30 parts by weight urea and 70—80 parts by weight water for two hours at 50°C, subsequently adding 100 parts by weight 30% formaldehyde and continuing the condensation for 12 hours at 30—50°C, thereafter heating up to 80°C and after-sulfitizing with 30—60 parts by weight sodium sulfite for 1 hour, then adding 400—800 parts by weight 45% lignin, sulfonate and stirring for 60 minutes at 50—80°C, thereafter adding 100—300 parts by weight 30% formaldehyde and condensing for 60 minutes at 50—80°C, then adding 20—50 parts by weight urea and condensing for 60 minutes at 50—80°C, adding water until the desired dry substance is obtained and adjusting the pH to 4—8.

**Revendications**

1. Fluidifiant pour béton et mortier, à base de ligninesulfonate, d'une part, d'un hydrocarbure aromatique sulfoné au noyau ou sulfométhylé, puis condensé, du groupe comprenant le phénol, le crésol et le naphthalène, d'autre part, caractérisé en ce que le mélange réactionnel des matières de base est condensé et sulfité.

2. Procédé de fabrication d'un fluidifiant pour béton et mortier, à base de ligninesulfonate, d'une part, et d'un hydrocarbure aromatique sulfoné au noyau, puis condensé, du groupe comprenant le phénol, le crésol et le naphthalène, d'autre part, caractérisé en ce que l'hydrocarbure aromatique rendu hydrosoluble par la sulfonation au noyau est ensuite condensé avec de l'urée et du formaldéhyde et en ce que ce condensat est condensé avec du ligninesulfonate en poursuivant l'addition d'urée et de formaldéhyde et est finalement sulfité avec du sulfite de sodium.

3. Procédé de fabrication d'un fluidifiant pour béton et mortier, à base de ligninesulfonate, d'une part, et d'un hydrocarbure aromatique sulfométhylé, puis condensé, du groupe comprenant le phénol, le crésol et le naphthalène, d'autre part, caractérisé en ce que l'hydrocarbure aromatique est sulfité par l'addition de sulfite de sodium et d'eau et est ensuite condensé avec du formaldéhyde, et en ce que ce condensat est condensé avec du ligninesulfonate, de l'urée et du formaldéhyde et est sulfité avec du sulfite de sodium.

4. Procédé de fabrication d'un fluidifiant pour béton et mortier à base de ligninesulfonate, d'une part, et d'un hydrocarbure aromatique sulfoné au noyau, puis condensé, du groupe comprenant le phénol, le crésol et le naphthalène, d'autre part, caractérisé en ce que l'hydrocarbure aromatique rendu hydrosoluble par la sulfonation au noyau est condensé ensuite avec de l'urée et du formaldéhyde, puis est sulfité et est condensé avec du formaldéhyde et de l'urée, du ligninesulfonate étant ajouté.

5. Procédé selon la revendication 4, caractérisé en ce qu'après la sulfitation de l'hydrocarbure aromatique sulfoné au noyau, on condense de nouveau avec du formaldéhyde, puis on ajoute du ligninesulfonate et on continue finalement à condenser avec de l'urée.

6. Procédé selon la revendication 4, caractérisé en ce qu'après la sulfitation de l'hydrocarbure aromatique sulfoné au noyau, on ajoute du ligninesulfonate, puis on condense de nouveau avec du formaldéhyde et on continue finalement à condenser avec de l'urée.

7. Procédé selon l'une quelconque des revendication 2 à 6, caractérisé en ce que du ligninesulfonate fermenté est ajouté.

8. Procédé selon l'une qielconque des revendications 2 à 6, caractérisé en ce que du ligninesulfonate non fermenté est ajouté.

9. Procédé selon l'une quelconque des revendications 2 à 5, caractérisé en ce qu'avant l'addition du ligninesulfonate, on détruit le sucre contenu dans celui-ci par oxydation alcaline.

10. Procédé selin l'une quelconque des revendications 2 à 9, caractérisé en ce que la lessive de ligninesulfonate contient, comme cation, un cation choisi dans le groupe comprenant le calcium, le magnésium, l'aluminium, l'ammonium et le sodium.

11. Procédé selon l'une quelconque des revendications 2 à 10, caractérisé en ce qu'il est réalisé principalement à un pH de 4 à 8.

12. Procédé selon la revendication 2, caractérisé en ce que 100 parties en poids de phénol sont sulfonées dans un autoclave pendant deux heures à 115°C avec 120 parties en poids d'acide sulfurique concentré; en ce qu'on refroidit à 50°C; en ce qu'on condense à 50°C pendant deux heures avec 30 parties en poids d'urée et 70 à 80 parties en poids d'eau; en ce qu'on ajoute 100 parties en poids de formaldéhyde à 30% et continue à condenser pendant 12 heures à 30—50°C; en ce qu'on ajoute 400 à 500 parties en poids de ligninesulfonate à 45% agite à 30—80°C pendant 30 minutes; en ce qu'on ajoute 20 à 50 parties en poids d'urée et condense à 30—80°C pendant 30 minutes; en ce qu'on ajoute 50 à 100 parties en poids de formaldéhyde à 30% et condense à 30—80°C pendant 30 minutes; et en ce qu'on ajoute finalement de l'eau jusqu'à l'obtention de la substance sèche désirée et règle le pH à 4—8.

13. Procédé selon la revendication 5, caractérisé en ce que 100 partiers en poids de phénol sont sulfonées dans un autoclave pendant deux heures à 115°C avec 120 parties en poids d'acide sulfurique concentré; en ce qu'on refroidit à 50°C et condense pendant deux heures à 50°C avec 30 parties en poids d'urée et 70 à 80 parties en poids d'eau; en ce qu'on ajoute 100 parties en poids de formaldéhyde à 30% et continue à condenser pendant 12 heures à 30—50°C; en ce qu'on chauffe ensuite à 80°C et sulfite pendant une heure avec 30 à 60 parties en poids de sulfite de sodium; en ce qu'on ajoute 100 à 300 parties en poids de formaldéhyde à 30% agite pendant 60 minutes à 50—80°C; en ce qu'on ajoute 400 à 800 parties en poids de ligninesulfonate à 45% et condense à 50—80°C pendant 60 minutes; en ce qu'on ajoute 20 à 50 parties en poids d'urée et condense à 50—80°C pendant 60 minutes; et en ce qu'on ajoute de l'eau jusqu'à l'obtention de la substance sèche désirée et règle le pH à 4—8.

14. Procédé selon la revendication 3, caractérisé en ce que 100 parties en poids de phénol et 40 parties en poids de sulfite de sodium sont sulfitées à 100°C pendant une heure avec 30 parties en poids d'eau; en ce qu'on ajoute 100 parties en poids de formaldéhyde à 30% et condense à 105°C pendant 2¹/₂ heures; en ce qu'on refroidit après distillation de 40 parties en poids d'eau et dilue ensuite; en ce qu'on ajoute 200 à 300 parties en poids de ligninesulfonate à 45% et agite pendant 30 minutes à 30—80°C; en ce qu'on ajoute 20 à 50 parties en poids d'urée et condense pendant 30 minutes à environ 30—80°C; en ce qu'on ajoute ensuite 50 à 100 parties en poids de formaldéhyde à 30%, condense pendant 30 minutes à 30—80°C et ajoute finalement 30 à 60 parties en poids de sulfite de sodium; en ce qu'on sulfite à 30—80°C pendant 30 minutes, ajoute de l'eau jusqu'à l'obtention de la substance sèche désirée et réglé le pH à 4—8.

15. Procédé selon la revendication 6, caractérisé en ce que 100 parties en poids de phénol sont sulfonées dans un autoclave pendant deux heures à 115°C avec 120 parties en poids d'acide sulfurique concentré; en ce qu'on refroidit à 50°C et condense pendant deux heures à 50°C avec 30 parties en poids d'urée et 70 à 80 parties en poids d'eau; en ce qu'on ajoute 100 parties en poids de formaldéhyde à 30% et continue à condenser pendant 12 heures à 30—50°C; en ce qu'on chauffe ensuite à 80°C et sulgite pendant une heure avec 30 à 60 parties en poids de sulfite de sodium; en ce qu'on ajoute 400 à 800 parties en poids de ligninesulfonate à 45% et agite pendant 60 minutes à 50—80°C; en ce qu'on ajoute 100 à 300 parties en poids de formaldéhyde à 30% condense à 50—80°C pendant 60 minutes; en ce qu'on ajoute 20 à 50 parties d'urée et condense pendant 60 minutes à 50–80°C; et en ce qu'on ajoute finalement de l'eau jusqu'à l'obtention de la substance sèche désirée et règle le pH à 4–8.